# EUROPEAN PATENT SPECIFICATION

(11) **EP 0 976 821 B1**
(45) Date of publication and mention of the grant of the patent: **18.02.2009**
(21) Application number: 98916796.0
(22) Date of filing: 20.04.1998
(51) Int. Cl.: C12M 1/34

(54) **EQUIPMENT, KIT AND METHOD FOR MICROBIOLOGICAL DIAGNOSIS**
DIAGNOSTISCHE MIKROBIOLOGISCHE TESTVORRICHTUNG UND VERFAHREN
EQUIPEMENT, JEU ET PROCEDE DE DIAGNOSTIC MICROBIOLOGIQUE

(30) Priority: 18.04.1997 CU 4597; 29.04.1997 CU 4897; 06.06.1997 CU 6597
(43) Date of publication of application: 02.02.2000
(60) Divisional application: 08159290.9
(73) Proprietor: CENTRO NACIONAL DE INVESTIGACIONES CIENTIFICAS, Ciudad de la Habana 12100 (CU)
(72) Inventor: CONTRERAS ALARCON, Orestes Rolando, Ciudad de la Habana 11400 (CU); ROURA CARMONA, Gloria, Marianao,Ciudad de la Habana 11400 (CU); NOVO MESEGUE, Francisco, Ciudad de la Habana 13600 (CU); HERNANDEZ RAMIREZ, Silvio, Ciudad de la Habana 19100 (CU); RAMIREZ FROMETA, Nardo, Playa,Ciudad de la Habana 11600 (CU); RAMIREZ MOLINA, Iván Manuel, Playa,Ciudad de la Habana 11600 (CU); TRAVIESO RUIZ, Fernando, Provincia Habana (CU); ZAYAS TAMAYO, Angela Mariana, Marianao,Ciudad de la Habana 11400 (CU); ROMAY PENABAD, Cheyla Edificio 2 Apartamento 208, La Lisa, Ciudad de la Habana 13500 (CU)
(74) Representative: Prins, Adrianus Willem
(86) International application number: PCT/CU1998/000004
(87) International publication number: WO 1998/047999

(56) References cited:
- EP-A- 0 304 406
- EP-A- 0 333 560
- WO-A-94/07123
- US-A- 3 609 040
- US-A- 3 712 144
- US-A- 5 345 395
- US-A- 5 637 082

## Description

### Technical Field.

The present invention relates to the field of microbiology, specifically to a method and equipment useful for rapid microbiological diagnosis, all of them having applications in human and veterinary clinic.

### Prior Art.

The microbiological diagnosis based on physical, chemical as well as biological methods, is an aspect that has been widely approached in the prior art.

The US patent 3,506,544 describes an electrochemical method for detecting bacteria through the measurement of the decrease in polarographic content of the oxygen which circulates through an electroanalytical cell that contains two different electrodes immersed in an inoculated culture medium. This system uses for its analysis great quantities culture medium (15-18 ml), thus making handling of the samples difficult on a routine level.

Another method that has been used to detect microbial growth is the voltaic cell principle, which is based on the use of an appropriate recipient with electrodes of noble metals and predetermined volumes, that generate a potential which drops at the moment of growth of the bacterium. In the specialized literature other equipments based on this principle have been described. For example, the equipment described by the patent GB 2142433 uses the same procedure, and uses a method of detection through the variation of the potential between electrodes which are in contact with fluid samples. This carries the implicit measurement of lower potentials with implementations of high impedance of the entrance that cause a change in measurable signal due to undesirable and unavoidable noises, that in most cases besides uses recipients with noble metals or non-recoverable golden layer recipients.

An efficient and simple method for detecting microbial growth is based on the measurement of conductimetric changes that occur due to microbial growth in a suitable culture medium. According to the relevant literature, ionic movements produce a signal which indicates the conductimetric value in a solution contained in a cell of the conductivity measurement.

It is known that conductivity cells have no total linear behavior in their baseline scale, and the measurement depends a lot on the temperature. In the patent US 4,482,967, a detector and a method to measure the conductivity that corrects these defects is described. This is an equipment of high accuracy and complexity and with special requirements to measure absolute values of conductivity in a gas chromatograph, with conventional cells and large volumes.

It is known that microbial growth can be detected in fluid samples by different methods, for example by using a turbidimetric method where the bacterial growth produces turbidity that is read by the system and compared with well-known standards.

This system requires optical conventional sensors, as well as the receiving container of the sample of optical quality, of complex design to work with samples that include visible solids that can be easily seen (For example, antibiotic discs). A great disadvantage of this system is the fact that it can not work with interfered samples, i.e. it requires optically homogeneous samples, besides the optic complexity that it bears. The patents US 3,852,532; US 3,895,661 and US 3,889,011 describe methods and apparatuses for that purpose based on these principles.

The patents US 4,021,120 and US 3,714,445 describe devices based on turbidimetric principle to measure the growth of microorganisms in liquid media. The patent US 4,021,120 describe a device to monitor the growth of microorganisms in a liquid medium that contains gas. Samples are taken from the recipient where the medium is, with the assistance with a pump that carries them to a degassing chamber with the purpose of eliminating gas bubbles. After this, the sample is introduced in a measurement chamber where a light ray passes through it. The light ray impinges on photoelectric cell, producing a current that is increased by an amplifier. This is an indicator of the growth of the microorganisms. The magnitude of this current will depend on the intensity of the light ray and it will be, at the same time, influenced by the turbidity of the medium. The sample then goes back to the recipient again when it is analyzed, with the assistance of another pump. This method of measurement, as well as the one described in the patent US 3,714,445 is very complex both optically and mechanically, while besides the measurement chamber also the pumps and the ducts of the transportation of the samples, should be frequently sterilized, this complexity makes its use in routine diagnostic methods difficult.

The patent GB 2 221 986 and patents US 3,819,278 and US 4,725,148 refer to turbidometers to measure directly the microbial growth using the same principle of previous methods. They present optical and mechanical complex systems and they require sterilization of their parts between each batch of microorganisms.

On the other hand, the patent US 3,832,532, also uses an optical conventional device that includes a cuvette of spectrophotometric quality, that in order to make measurements using antibiotic discs, includes in its design a bilobal interconnected chamber, in such a way that once the incubation is finished, the liquid must pass to the other chamber in order to perform the measurement, and thereby avoiding the presence of the antibiotic disc during the reading operation. The proposed solution of this invention presents a great complexity not only operatively but also technically and it has economical implications.

The present trends of microbiology are directed towards the search of procedures that allow a quick identification of the microorganisms (between 2-4 hours) in biological samples. To achieve this, different strategies have- being used, among them the use of specific enzymatic markers.

According to the state of the art, the majority of the biological samples cannot be used directly, necessitating the prior isolation and growth of the microorganism to be identified. This takes a lot of time, and it is impossible to obtain results before 24-48 hours after receiving the samples in the lab.

The infections of the urinary tract are considered one of the most frequent among infections diseases.

The classic techniques for detecting bacterial infections in the urine require a cultivation in plates, which requires at least 24 hours to remove all negative samples and to select the positive ones.

It is known that only 20% of the urine samples that arrive at the lab are positives and from these, 70% correspond to infections by E. coli. That is why the identification of E.coli allows a considerable saving of time and resources, because only 30% of positive samples would be isolated for their subsequent identification.

According to the state of the art, the identification of E.coli is carried out mainly by two specific enzymatic markers of this bacterium, β-D-glucuronidase and tryptophanase enzymes, through Indol formation (Kovacks, N. Eine vereinfachte Methode zum der Nachweis der Indolbildung durch Bakterien. Z. Immunitatsforsch., 55; 311-315, 1928). 94% of all E.coli, a few Salmonellas and Shigellas show positive reaction towards β-D-glucuronidase. Indol formation is positive for 99% of all E.coli. Thus, the combination of both tests allows unmistakable identification of this microorganism.

At present, different tests are being commercialized, like BACTIDENT-E.coli and different culture media like FLUOROCULT-MUG, (both from MERCK DIAGNOSTICA). They are based on the above principle. However, for their utilization a prior isolation of the microorganism is necessary to obtain an isolated colony, to accomplish the BACTIDENT identification, or the sample is inoculated in the culture medium and 24 hours are needed to obtain the isolated colonies and to be able to detect the changes associated with the transformation of the specific substrates (FLUOROCULT-MUG).

In the patent application No. WO 95/03424, a solid culture medium for simultaneous detection of coliform bacteria and/or E.coli in water samples and in foods is reported. For this, 24 hours of incubation is required after inoculating the plate with the sample to be evaluated. Similar procedures are followed in the Diagnostic Kit URILINE ID and the culture medium CPS ID, both from BIOMÉRIEUX, France. The incubation of the samples on solid medium during 24 hours before identifying the microorganism is necessary in both cases.

The patent application No. WO 80/02433 refers to a procedure to identify bacteria through the combination of different tests to determine 26 bacterial enzymes; among them β-D-glucuronidase and Tryptophanase are useful to identify E.coli.

In this invention, bacteria should be isolated from the clinical specimens before their identification.

### Disclosure of the invention.

The objective of the present invention is to provide a system that allows to detect microbial growth early, in urine samples obtained directly from animals in which the detection of the growth of microorganisms is required through the use of micro-samples. This system is based on the detection of turbidimetric changes in a culture medium, produced by the growing microorganism. This system includes a method and equipment designed for this purpose.

The method for microbial diagnosis in urine according to the present invention comprises the following steps:
a) An aliquot of a urine sample directly obtained from its source is innoculated in a glass vial containing culture medium, a polymer and substrates MU-β-D-glucuronide and L-tryptophane, both solubilized in potassium phosphate 50 mM, pH 7 to 7.5,
b) The turbidity of said vial is determined to time = 0;
c) The vial is incubated between 2 and 6 hours to a temperature between 35 and 37° C;
d) The growth index, is determined between time 0 and the selected time of incubation and samples positive are discriminated from negative ones in dependence of the increase of the turbidity, being positives those samples with increases higher than. 0.08 McFarland units, from which aliquots are taken for further steps;
e) an aliquot of a positive sample is taken from step d) and it is transferred to a new glass vial containing fresh culture medium, which is dispensed in a volume of 200 µl in each microwell of a nontransparent strip containing two free positions for negative and positive controls, which are filled with only culture medium and inoculated culture medium respectively, and 10 to 22 positions in which antibiotic discs are organized, and it is incubated between 3 to 4 hours to a temperature between 35 and 37° C;
f) the strip is read by placing a turbidimetric reader of microflow, which is fed by a peristaltic pump, in each microwell following the instructions emitted by a computer program package for data acquisition and processing and creation of databases designed for that purpose, wherein the turbidimetric reader of microflow comprises a photoemitter, a photodetector, and a measurement chamber, wherein said measurement chamber is composed of a plastic tube introduced in a glass capillary, the measurement chamber being illuminated by a light emitted from said photoemitter and passing through said measurement chamber;
g) from the density values obtained, the growth index is calculated in the controls, and the inhibitions percentages are calculated for each sample for each antibiotic;
h) according to the inhibition level show by the samples, the criteria of sensitivity, resistant or intermediate are adjudicated, among inhibition values in a range of 60 to 100%, wherein samples with inhibition percentage smaller than 60% can be considered resistant, those showing values between 60 to 80% can be considered sensitive in an intermediate level and those which are inhibited between 80 and 100% are considered sensitive to the antibiotic tested;
i) an aliquot taken from the step d) is submitted to a source of U.V. light, in order to detect the fluoresce generated due to 4-methylumbeliferone liberation, and developing the Indol formation using a modified Kovacks reagent, constituted by 2 g of paradimethylaminobenzaldehyde diluted in ethanol and 20 ml of concentrated hydrochloric acid, for detection of MU- beta -D-glucuronidase and tryptophanase enzymatic activity produced by E.coli;
j) for those samples wherein E.coli was not identified in the step i), the aliquot previously obtained in the step d) is submitted to the traditional process of isolation and identification.

The equipement of the present invention suitable for carrying out antibiogram determination in steps f) to h) of the method according to the present invention comprises :
(1) a turbidimetric reader of microflow (1) comprising a photoemitter (12), a photodetector (13), and a measurement chamber (9 and 10), wherein said measurement chamber is composed of a plastic tube (9) introduced in a glass capillary (10), the measurement chamber being illuminated by a light emitted from said photoemitter and passing through said measurement chamber;
(2) a nontransparent strip containing microwells;
(3) a peristaltic pump (2) for circulating a sample through the measurement chamber of the turbidimetric reader of microflow; and
(4) a computer (5) having a program package for data acquisition and processing and creation of databases;
   wherein
   the turbidimetric reader of microflow is connected to the computer by an interface card and
   said turbidimetric reader of microflow includes a mouth part (8) suitable for introduction in a sample in a microwell of the nontransparent strip to be tested by circulating a sample through said measurement chamber by action of said peristaltic pump.

The novelty of the present technical solution is that it allows detecting infected samples, directly obtained from the species which produce them.

Additionally, among other applications, the present invention permits getting the sensitivity scheme to the different antibiotics of microorganisms using previously isolated colonies or positive samples of urocultures, in this case saving the time required for isolation and purification processes.

In the particular case of urinary tract infections, the present invention allows to discriminate positive samples from negative ones using direct urine samples, contaminated or not with other germs. It can also include the simultaneous identification of those infected samples, specifically with E.coli bacterium.

With the system of the invention, the results based on more than 1000 tested samples have shown a 95% of concordance with total count of viable cells in CLED culture medium. This method is conventionally used to detect urinary tract infection.

In the antibiogram determination, the correspondence with Bauer-Kirby method has been established as 92.4%, major errors 1,3%, and very mayor errors 0.4%.

The present invention provides useful information related to clinical microbiological diagnosis in a short period of time. This information is very important to prevent the inadequate use of antibiotics, the development of microbial resistance, long hospital stays and death in the case of serious infections.

The system is characterized by its celerity. In a period of four hours the urine infection can be determined and on positive samples reliable antibiogram results could be obtained.

At the same time, this system is in a distinguished manner highly precise. The obtained results can be checked as often as necessary.

From a social point of view, the system has great importance due to the possibility of providing antibiotics in a rational and convenient way, avoiding long hospital stays. From an ecological point of view, the invention similarly restrains the development of bacterial resistance.

It is a highly flexible system that allows to adapt the information of the program that it uses to the necessities or requirements of the users. The system offers the possibility to change the set of antibiotics used according to the particular needs.

The system of the present invention consists of an equipment and a method designed for rapid microbiological diagnosis, which can be applied in the human and veterinary clinic.

The equipment has been designed to work with a great amount of samples and it uses an operative program, and a man-machine interface that is very functional. It also provides an audiovisual alarm to arrange the reading/lecture and avoid operational mistakes. The module of measurement of this equipment can be inserted in the free slot of a computer machine.

The equipment of the present invention can comprise the following devices:
- A control module which is incorporated in a personal computer.
- An interface card.
- A peristaltic pump.
- A sensor.
- A calibrator.
- A printer.
- A UV lamp, optionally.

The personal computer should have the following properties:
- IBM Compatible, 386/486, 25-66 MHz.
- Memory RAM 1 Megabyte minimum.
- Hard Disc: 40 Megabyte minimum.
- Tower of Floppy Disc 3 1/2", optional.
- Display SVGA Color.
- Keyboard, Mouse and Printer.

The peristaltic pump has been designed to produce the circulation of the samples through the sensor. It has a manually adjustable cassette that allows a regulated and uninterrupted flow, which may be used independently or incorporated into the system. The pump uses a flow of 2.0 - 2.6 ml/minute and it is fed optionally by 220 Vac or 12 Vdc. Its potential consumption is 0.5 watts.

The sensor is composed by a reader of continuous microflow which is used to detect changes in turbidity, due to microbial growth in previously prepared samples coming from different sources, in very small volumes (up to 200 microliters), in movement, i.e. in moving liquid, where samples can be or not interfered. Using the present invention it is not necessary "to clean" the reader of flow during measurements. Thus discrete samples can be measured or by continuous way. This measurement is not interfered by turbidimetric variations of culture medium as a consequence of the changes of the samples. However it allows to detect small variations medium turbidity due to microbial growth. The control of the temperature is not required for the measurements, and it is possible to make an undetermined number of measurements, of different samples, from different sources and microbial cell concentration.

Besides, this sensor is distinguished by its calibration which is automatic and to a continuous flow of 2 - 2.6 ml/minute. Optic range of measurement is 0.00 - 2.00 McFarland units.

In the present invention a calibrator is used which is based on nephelometric techniques and it follows the McFarland scale. It is composed of a direct light source and a photosensor and it is adjusted through a program. The calibrator's function is to measure the turbidity in Mueller-Hinton liquid medium up to 2.0 McFarland units. Energy requirement is 5 VDC and its consumption is 50 ma.

The UV lamp that is coupled optionally to the equipment allows identification of the bacterium E.coli in tested samples. The program offers a friendly man-machine interface, it is easy to handle and allows the selection of different options by Bar-menu or function keyboard, combined with the use of icons, as well as the automatic storing of data.

It uses a structured interrogative language (SQL) for obtaining information and an external utilities 'BACKUP' which makes security copies.

The program has an alarm system to be used in the case of infringement of the entrance condition, an audiovisual alarm to control the reading time of each sample, as well as other utilities for technical maintenance.

The distinctive essential features of the equipment of this invention are the turbidimetric static minireader, the microflow sensor which is fed by the peristaltic pump, and their connection to a microcomputer with a program package for acquisition, processing and creation of data bases, to be used in generating necessary reports.

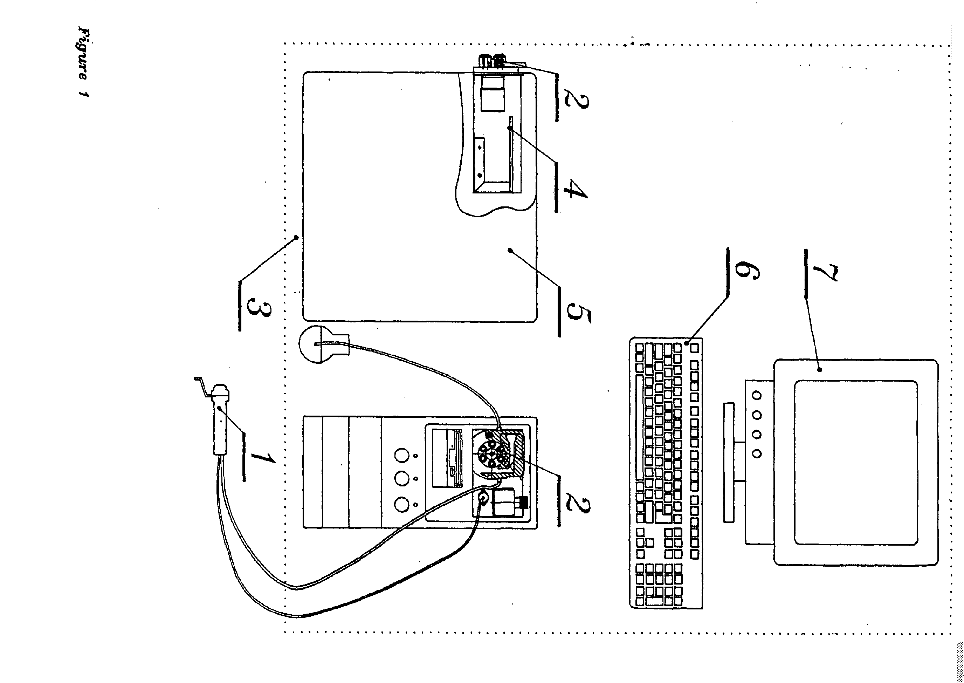

Figure 1 shows the integral scheme of the equipment of the present invention, in a general overview. As it is shown, this equipment is based on a turbidimetric reader of microflow (1), which is fed by a peristaltic pump (2) and adjusted to an electronic equipment of high sensibility (3) that detects turbidimetric changes of (1) through a measurement method which allows using a group of algorithms to detect small turbidimetric variations and properly process obtained data. It is formed by a turbidimetric measurement circuit (4) connected through an interface card to a central processing unit (5). This unit receives all keyboard commands (6) and delivers the results in a display (7). It can detect small variations in turbidity that occur in the culture-inoculated medium in the sample to be tested.

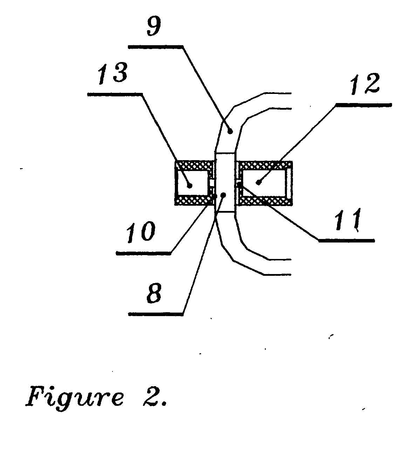

In Figure 2 is shown a detailed scheme of the internal structure of the turbidimetric reader (1) where the variations in turbidity are detected. The procedure for measuring is very simple: the small mouth of the reader (8) is introduced in the sample to be tested which then circulates through it with help of the peristaltic pump (2) of Figure 1. This peristaltic pump works all the time required to carry out the measurements. First of all the presence of the sample is detected by obtaining a voltage value which exceeds a pre-designated threshold and after a determined period of time the measurement is done and so on. As the peristaltic pump (2) of Figure 1 is kept working, there is a period of time between each measurement during which air circulate through the turbidimetric reader (1). This period of time is considered as the moment in which the turbidimetric reader (1) is cleaned, and means that the additional process of washing is not necessary in order to sterilize all part of the reader.

The measurement chamber is composed by a plastic tube (9) introduced in a glass capillary (10). The light pass (11) is the orifice diameter through which the light coming from the photoemitter (12) should cross over to arrive to the measurement chamber (9 and 10). The intensity of the luminous radiation, transmitted through the measurement chamber (9 and 10) will depend on the level of turbidity of the sample and it is measured by a photodetector (13). The radiation produced by the photoemitter (12), is stabilized by means of an electronic loop of conventional automatic control.

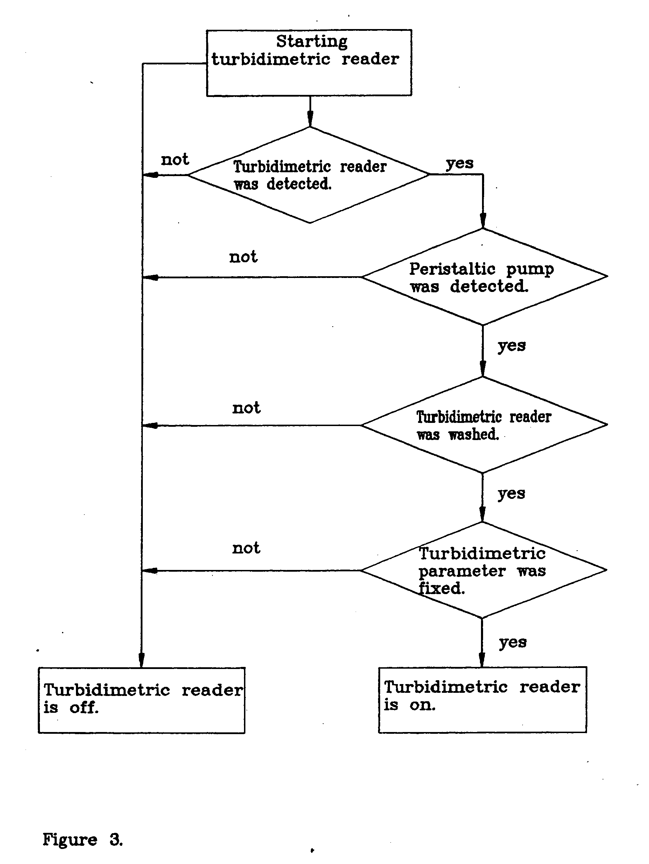

Figure 3 shows the turbidimetric reader working through a diagram of flow. Firstly, the presence of the turbidimetric reader is verified by means of the subroutine of detection and if it is present, then the existence of the peristaltic pump is checked which is necessary for the working of the turbidimetric reader and for the cleaning execution subroutine, besides establishing the required working flow. The cleaning subroutine is of significant importance because the reader's parameters depend on the cleanliness of the measurement chamber. This aspect influences its useful lifespan.

Once all the working parameters has been regulated, the turbidimetric reader will be ready. Any subroutine that is infringed will disable the reader from functioning. The main application of this device is oriented towards the antibiogram determination of the sample, (the susceptibility of the microorganisms to the antibiotics), which is achieved in a period of time between 2 and 6 hours, supported by a diagnostic kit designed for these purposes.

The other device which is incorporated into the equipment of the present invention is the static turbidimetric minireader mentioned before. It detects turbidity changes due to microbial growth of the sample that was used in a glass vial that contains liquid culture medium, which is one of the components of the diagnostic kit of this invention. This vial is exactly adapted to the reading shaft of the equipment. The device is adjusted to use the mentioned vials for the direct reading of the sample, which means that special cuvets are not necessary to execute the reading,which makes its use in routine diagnostic media possible. The mentioned device is used to calibrate the inoculum that is used in the diagnostic kit for detecting the antibiogram. It reports turbidity in McFarland units, according to latex patterns of NCCLS standards in a established range of measurement (0 - 4,0 McFarland units).

The diagnostic kit described herein is composed of a nephelometric vial of 8 ml volume that contains 4,5 ml of culture medium, which may be a glass vial of autoclaveable borosilicate with a plastic cover.

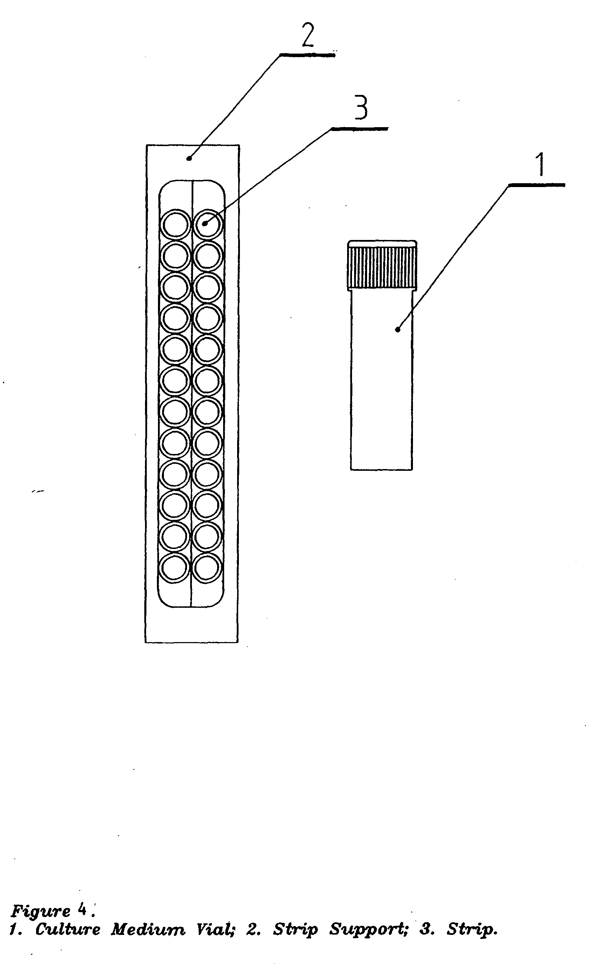

Figure 4 shows the components of the diagnostic kit, constituted by a vial containing culture medium and the polymer, the strip support and the strip used for antibiogram determination of the sample to be tested.

Modified Mueller-Hinton Broth OXOID sterile, pH of 7.4 ± 0.2 is used as culture medium in order to follow microbial growth, which includes additionally a polymer.

The diagnostic kit used for detecting an antibiogram in a sample is characterized by the use of antibiotic discs commercially available. They can be used according to schemes that may change according to any requirement. Antibiotic discs are organized into non-transparent strips containing two free positions for negative and positive controls, which are filled with only culture medium and inoculated culture medium respectively. They are used to calculate the growth index in the samples to be tested. Furthermore, there are other 10 to 22 positions where antibiotic discs could be placed.

The program created for these purposes, allows the introduction of these changes in the acquisition and edition processes. It means that the kit has high flexibility and permits adaptations according to different necessities.

A polymer; that could be any linear polysaccharide of structural formula CH3-CH3-Ch3-N or similar to this one, with molecular weight between 50,000 and 150,000, is added to the glass vial aforementioned containing culture medium for dilution, which form part of the diagnostic kit of the present invention. The incorporation of this polymer to the culture medium at the concentration in the range between 0.05 and 1%, allows to eliminate the inhibitor effect of catabolic products accompanying the sample inoculum to be tested, thus greater growth indexes of the infection involved bacteria are obtained in a smaller period of time, in comparison with the required time when the same culture medium is used without said polymer. This new element in the diagnostic kit reduces false sensitive results obtained in susceptibility studies, and at the same time improves the correspondence with the Kirby-Bauer reference method (Bauer, A. W.; Kirby, W. M. M.; Sherris, J. C. and Turck, M. An. J. Clin. Pathol. 1966, 45, page 493-496).

Considering that this polymer is only metabolizable by a limited number of microorganisms which are generally not found in those analyses where the present system is applied, it is inferred that the bacterial growth promoting effect is brought about by an inhibition of the inhibiting agents which are present in the culture medium. For this reason said polymer should act by absorbing the catabolites which are involuntarily incorporated together with the inoculum that is analyzed. Besides, it has been observed that once these catabolites are neutralized by the mentioned polymer during the microorganism growth measurement process, the bactericidal activity of tested antibiotics is more specific.

One of the advantages of the present invention is the fact that the system allows for the diagnosis of not only previously isolated strains but also direct samples of urine.

First of all, the referred sample is placed in the glass vial containing the polymer and the culture medium, immediately thereafter the turbidity is determined (t0) and this value, as well as the reading time according to the adjudicated number of each sample, is stored by the employed program. After that, the mentioned vial is incubated between 2 to 5 hours at a temperature between 35 and 37°C. At the end of the incubation, the system emits, according to the routine structured, a beep alarm sound and screen warning indicating that the sample should be read again. Those samples that show increments higher than 0.08 McFarland units, are considered as positive samples.

The system of the present invention allows, on positive samples once they are detected, the determination of their antibiogram in a very rapid way. For that purpose, an aliquot of the sample is taken and transferred to a new dilution vial that contains fresh culture medium, which is distributed in the strip where two controls (positive and negative) and from 10 to 22 antibiotic discs are found. After a 4 hours incubation period at a temperature of 37°C, the strip is read, by putting the microflowsensor in each microwell, following the instructions given by the program, which selects the moment at which each measurement in series is carried out. Thus, the interference of the previous reading is eliminated. From the obtained density values, the growth indexes are calculated (in the controls), as well as the inhibition percentage for each sample by each antibiotic. According to the inhibition level show by the samples, the criteria of sensitive, resistant or intermediate are adjudicated, among inhibition values in a range of 60 to 100%. This means that those samples with inhibition percentage smaller than 60% can be considered resistant, those showing values between 60 to 80% can be considered sensitive on an intermediate level and those which are inhibited between 80 and 100% are considered sensitive to the antibiotic tested.

Each result is checked in order to value if it is between a minimum or maximum admissible growth (satisfactory antibiogram). The obtained results and edit data of each sample, automatically pass to create the corresponding data bases.

As was explained before, the invention permits work with direct samples in liquid medium, read in commercial vials and afterwards execute antibiogram in positive samples. This whole process is carried out in a period of time of less than 9 hours, avoiding thereby prior steps of isolation and purification of the samples and obtaining sensitivity levels higher than 90%. and obtaining sensitivity levels higher than 90%.

The proposed system also allows to cope with interferences generated due to contaminated samples as well as infections caused by more than one germ. The contaminated interference has been overcome by adjusting the signal strength and the time for detecting infection levels that are internationally accepted (>100,000 ufc/ml), this way, contaminated non-infected, samples are not included for further processing, which generally have low bacterial level (< 1000 ufc/ml), which gives the opportunity of detecting contaminated samples only when they are infected. In this particular case, taking into account the fact that the contaminating species are generally saprophytes, that according to gram reaction, are sensitive to all antibiotics, it is evident that they cannot interfere in the detection of the resistance scheme of infected strains. These constitute distinctive characteristics of this invention.

In relation with infections produced by more than one germ, two situations could be presented: in the first case one of the infecting germs could predominate due to a larger specific growth rate, after the minimum time of incubation , in this case the antibiogram will be accepted. In the second case, where both germs grow at the same rate, the antibiogram could show one effective antibiotic for both, or a scheme of absolute resistance could be presented. In the last case, a new antibiotic could be tested or the sample should pass through isolation and purification procedures.

This conjunct of analysis and rapid solutions for each particular situation is possible thanks to the application of the reading concept in direct samples with a high level of interference, in a short and fixed time, aspects that characterize and distinguish the system of the present invention.

In addition to the urinary tract infection detection report and infecting bacteria susceptibility pattern, bacterial identification should be incorporated, in order to produce a complete report. Aiming to achieve this purpose, to the mentioned vial with culture medium and polymer, used for detection for urinary infection, two substrates that allow rapid identification of E. coli bacteria in urine, could be added, as has been stated before, in the same detection vial.

In the present invention, a designed culture medium that could be used in the proposed system allows detection of E.coli in 4-6 hours incubation time, from infected urine samples tested.

Proposed culture medium used in the present invention, include per liter, in addition to the conventional nutritional bases included in Oxoid Mueller Hinton culture medium : (Meet infusion, 300 mg; casein hydrolyzate, 17 g. and starch, 1,5 g.), the substrates MU-β-glucoronid (0,1 g), L-tryptophane (1 g), and the polymer used as de-repression agent (1 g). All those components are dissolved in potassium phosphate buffer 50 mM, and the medium pH is adjusted between 7-7.5. After that the medium is distributed in 4.5 ml volume in vials, that finally are sterilized by autoclave during 20 minutes at 121° C.

Mentioned substrates, are useful for detection of MU-ß-D-glucuronidase and tryptophanase enzymatic activity, produced by E.coli. For the last enzyme, Indol detection is needed, so after bacterial growth is obtained in the medium, an auxiliary reactive is added for activity develop (modified Kovacks chemical reactive) ;which formula is : paradimethylamino benzaldehyde (2g), ethanol and concentrated chlorhydric acid (20 ml).

The product of the enzymes-substrate interaction is detected, as a first step by exposing the vials with a turbidity increment (positives), to a UV light source, (Item that optionally could be included to the equipment of present invention), for florescence detection that is generated from 4-methilumbeliferone liberated. As a second step, Indol production is tested in the same vial, by addition of Kovacks modified reactive.

### EXAMPLES:

### Example 1. Put on the equipment:

Just 15 minutes before start lectures, the equipment is on.

Once computer is activate, the auto-execution program drives directly to the program designed for the execution of all process, in this way initially, will test the existence and integrity of data base and; checking functioning capacity of each one of measurement module components : sensor, inoculum calibrator and peristaltic pump. Also, it will check electronic key existence, that is coupled to a parallel port of the computer. Program will inform about any mistake detected in any of system elements and will disable related options. If data base had been not created , will be created if the user takes this decision. If there is no any data base, it will be created if the user decides it.

### Example 2. Sample preparation for uroculture tests:

Uroculture is a well known test, used for screen urine samples infection. Procedure stars by optical density measuring immediately after vials had been inoculated (T0h), followed by a second measurement 4 hours after incubation (T4h).

The measurement is made photomethrically by inoculum calibrator utilization. The following steps show the whole procedure:
1. A 4.5 ml sterile culture medium vial is inoculated with 500 µl of urine.
2. Initial turbidity is measured in McFarland units using inoculum calibrator (T0h).
3. Sample is incubated at 37°C during 4 hours, once ended, the equipment emit a sound alarm for each sample.
4. A measurement of turbidity of inoculated vial is made after 4 hours of incubation (T4h), and according to the level of increment detected, samples are classified as positive, negative or doubtful. In last case, sample should be checked after one additional hour of incubation for screen probable positivity. Also in patients with antecedents of renal disease, it is recommended the same procedure as before (T5h).

According to needs, when all measurements had been done, edition of each case could take place, and stored in system data base. If automatic printer option is activated and printer is ready, cases will be automatically printed. Samples classified as positive are ready for antibiogram detection (directly).

### Example 3. Procedure for antibiogram

For antibiogram, strips of 12 to 24 microwells are used. The two first are for positive (C+) and negative (C-) controls, respectively ;antibiotic discs are placed in the others microwells.

Strip inoculation is done by sample distribution in the first microwell and those with antibiotic discs. The second microwell is filled with sterile culture medium.

**Example 4.** Inoculum preparation.
- From a pure strain.
   If inoculum is prepared from a pure strain, following steps should be executed :
   1. Three or four colonies from a fresh culture medium (18-24 h) are taken and added to sterile 4.5 ml of Mueller Hinton broth reinforced with linear polymer, molecular weight 50,000, till a 0.5 McFarland scale unit is obtained, by mininephelometer testing. Option that appear in principal menu (McFarland).
   2. From this cell concentration 150µl are added to 4.5 ml sterile culture medium placed in other vial, and homogenized by stir.
   3. From last dilution 200 µl are distributed in the strip for antibiogram, in the microwells used for positive control (C+) and those with antibiotic discs.
   4. In the second microwell, used for negative control (C-), 200µl of sterile culture medium are distributed.
   5. Once finished strip is sealed and incubated at 37°C during 4 hours.
   6. Strip is taken from incubator and after 10 minutes room temperate, lectures start. It is recommended to homogenized the strip by manual or mechanical stir.
   7. The strip is opened and the reading begins.

   It is a requirement to read the antibiogram that the difference between (C +) and (C -) achieves a given value defined by the Admissible Minimal Growth. If in presence of the values next to the differentials established by this parameter, are detected in each one of the proven antibiotics, inhibition levels homogeneously low (Antibiogram not reliable), the antibiogram must be repeated. In these cases it is recommended to reduce until reaching 30% the volume of the inoculum used previously.
- If the inoculum is prepared from a positive urocultivo, to accomplish its antibiogram, it is accurate to make a previous dilution of the sample until reaching 0.5 of the McFarland scale and once reached the turbidity wished is continued the process from point 2 described previously for the obtainment of the inoculum from a pure strain. In those cases in which the uroculture will be detected as positive in 4 hours and has inferior values to 0.5 of the McFarland scale, this must be incubated until achieve said value or it can be spread in a culture medium to election, to be worked on the following day, since this can be had due to:
   1. Low load of non infecting microorganism.
   2. Patient under the action of antimicrobial therapy.
- When the inoculum is prepared from positive hemocultures (of 18 to 24 hours of incubation) should be accomplished the following previous steps:
   - Extraction of the superior phase of the hemoculture (supernatant) 200 µl and to add them in 4,5 ml of culture medium Mueller-Hinton + Pol-10.
   - To read the turbidity in the mininephelometre in the option McFarland of the Main Menu, and to monitor until achieve values of 0.5-0.7 of such scale.
   - The process of point 2 described previously is continued as same as for the obtainment of the inoculum of a pure strain.

### Example 5. Program Applications.

The present disclosure supplies a set of full interactive programs to carry out the Antibiogram and Uroculture tests. Data obtained in each test can be stored in the database for further processing. The user is guided inside an easy to operate environment that does not require previous experience in computer operation.

Each test is treated in independent menus that build up a Main Menu.

To access to any of the system's options:
1. Press the key that corresponds to the underlined letter in the desired icon.
   - Place the mouse pointer over the selected icon and press its left button.

To cancel the selected option, press the "ESC" key or use the mouse to select "Return" or "Cancel" to return to the previous option or cancel the operation.

### Main Menu.

Once the system initialization is finished, the program will show the Main Menu, which is the starting point for all the operations that can be done. The main window of the menu is composed of three basic panels:
- Central panel that shows the icons which activate the main options of the program
- Upper panel which shows information related to the system's version, date and time, current menu and a brief explanation of how to operate to choose a new option. The upper panel supplies two special options found to its right most corner:
   **Exit:** Used to stop execution of the program at any moment, as long as a measurement process or a critical operation is not being executed. It is recommended to always use this option. **DO NOT TURN OFF THE COMPUTER** while the program is running to avoid eventual software conflicts.
   **SCR. S.:** Allows the user to activate, at choice, the system's Screen Saver when the instrument is going to be left in a Stand By condition to avoid screen damages that can occur when a steady image is left constantly during long periods of time.
      Note: This option will be activated automatically if the system is left inactive for a certain time. To return to normal screen, move the mouse or press any key.
- Bottom Panel: Shows information about the uroculture test. In case some of them are waiting to be read, the amount and the time at which the one heading the list, sorted by the "next reading" option, should be read. The bottom panel also shows whether the system's alarm for uroculture reading is activated or not.

### Main Menu options:

The principal menu is integrated by a set of icons that contain drawings related to the operation that is performed when these are activated. A black triangle noting toward the right in the options "URO and ATB" is an indicator that notifies that upon activating one of them will be shown a submenu with additional options.

Nine icons define the available options within main menu, from where are controlled all the actions of the system. These are:
URO: Activates the icons menu of uroculture.
ATB: Activates the icons menu of the antibiogram.
McFarland: Permits to measure the content of the vial in the calibration unit (McFarland scale).
Data base: Permits to access to the data base of antibiograms or urocultures.
Options: To change the configuration of the system.
Information: Shows administrative information of the system.
DIRAMIC: Offers the address of the producing of the system.
Help: Gives information on the current option.
Exit: To abandon the system.
Menu of Urocultures
Uro: Menu of Urocultures

Upon selecting the **URO** icon of the Main Menu, a sub-menu with a new set of options will be provided for the measurements of Urocultures. Within this menu the following actions are provided.

### Description of the options of the Urocultures Menu:

***Read uros:***
   Procedure for the reading of urocultures. Upon activating this option, a list of the uroculture cases that are waiting to be read is displayed, where the consecutive case number, the time that must elapse between readings and the date and time of the next reading are shown. If the list is empty, no cases are pending.
   To select a specific case of the list, press the left mouse button twice at a relative high speed (or press the keyboard's space bar) while pointing the case. The selections will then appear with a check mark to its side and the majority of the operations will be effected only to them.
   The "READ URO" selection will provide the following options:
   - **New uros:** Allows to read the T0h (first reading) of the cases of uroculture. If the McFarland calibrator has not been previously compensated, the program will ask the operator to place a sterile culture medium flask in the calibrator's well in order to set the zero reference before adding new cases. Once the McFarland calibrator's compensation is done, a message window will inform the operator about its results. A "Compensation Factor" will evaluate the calibrator's performance. Any value below 2.5 will be accepted. However, values beyond 2.0 might be an indicator of dirt in the measuring well. In such cases, try cleaning the well with a small piece of cotton fabric damped in Ethylic Alcohol. Subsequently, a window will be shown to select among reading, editing, or changing the time that must elapse between readings (T0-TFh). This last option must be set before the second reading is done.
   - **Re-read:** Allows to repeat the reading of time T0h of a sample. This option is useful to rectify an error in the reading of one of the cases. When activating this option a window with the same possibilities that the "New uros" option will be shown plus a new choice for jumping to the "Next" case without modifying the current one.
   - **Edit:** By means of this option, a form with the general data of the patient can be filled. Another group of fields gathers information of interest for the laboratory. In order to save the data that has been typed press the left mouse button over the desired option. Alternatively, use the "RETURN" key to accept or the "ESC" key to abort the edition process.

While editing several cases, the option "Next" will become available. With this option the edition of the current case is abandoned and the action jumps to the next case. If the option "OK" is selected, the data set will be stored and the next case will be automatically presented for edition.

A field marked by a black triangle pointing down indicates that when this field is selected, a pre-edited list will appear in order to select the desired text. An intelligent search is possible by typing the first letters of the searched word. This edition style is used to standardize the text corresponding to important fields avoiding spelling mistakes that could confuse a "Search" process. Words in this list can be deleted, modified or added, but if a text element of the list is modified, the complete database will be modified accordingly.

To jump from one field to another within the edition process, press the TAB key or simply press the left mouse button on the desired field.

If several cases are simultaneously selected for edition, a "Next" option will become available. This option ignores the changes made to the current case and jumps to the following. On the contrary, if the option "OK" is selected the current data will be saved to the database before the next case is presented for edition.
- **T0-TFh:** Allows the user to set the time that will elapse between the first and the subsequent reading. This option will be executed for all the selected cases whenever it is possible.
   *Note: If the time that has been set is shorter than the time that has already elapsed, the system will send an error message.*
- **Cancel:** Allows the user to delete undesired pending cases. This option will be executed for all the selected cases.
- **Read TFh:** Allows to execute the final reading of a uroculture in order to obtain the result of the test. Upon activation of this option, a window will appear displaying the general data of the case and its classification as "Positive, Negative or Doubtful." To accept this reading select "Read." If the option "Next" is selected instead of "Read", the results of the current case will not be considered as final and the action will jump to the next selected case. This option is used when the operator decides to increase the time between readings in order study the evolution of a specific sample.
- **Select:** Allows to select all the cases in the list.
- **Sort:** Arranges the list by the consecutive number or by the time of next reading. This option is useful when it is desired to sort the cases according to the time when they are to be read, which does not always coincide with the consecutive order.
- **Print:** Supplies a dialog to the user in order to configure the output of data to a suitable printer. The following options can be selected:
   - **Printer:** Allows the setting of a specific type of printer:
      - Epson: For standard dot matrix printers.
      - HP Laserjet: For Laser printers.
      - Adobe Script: For Postscript printers.
      - ASCII: To write data to an ASCII file. You can specify the name and path of the text file where data is stored in case this option is selected.
      - Cancel: Returns to the previous option.
   - **Copies:** Number of copies that will be printed.
   - **Lines/Page:** Number of lines of text in each page.
   - **Print to:** Allows to select the output port of the Computer where the printer is installed, usually LPT1, LPT2, LPT3 or LPT4.
- **Return:** Returns to the previous option.
   The available commands inside this option are:
   - **OK**: Starts printing. After activating this option an information message about printing choices will be displayed. The possibility of canceling the printing is included.
   - **Cancel:** Cancels the operation.
   - **Return:** Transfers the control to the UROCULTURE MENU.

### Report:

Generates a report with the results of the uroculture test. This report may be printed.

### McFarland:

Allows the measurement of microbial growth in McFarland units according to NCCLS standards, taking as zero growth reference a non inoculated sterile culture medium. The readout is shown in a graphical scale and digital mode. The second figure of the digital values should be seen and understood only as a reference of the sample's trend. Additionally, an approximate cell count, obtained by mathematical calculations in accordance with NCCLS standards, is also supplied.

To perform the measurements, the outside surface of the flasks used in the calibrator must be clean and free from scratches.

In order to increase precision, flasks that are measured in the McFarland calibrator should be marked in such a way that its position can be easily repeated each time the flask is read. The mark should always be visible to the operator.

Two commands are available within this window:
- **Compensate:** This action sets, at user's choice, the 0 McFarland reference for microbial growth readings of the sample flasks. It can be useful if a different batch of culture medium is going to be used after the calibrator was compensated. The process is achieved by placing a flask with an non-inoculated culture medium in the measuring well of the McFarland calibrator.
- **Return:** Returns to the previous option.

### Database:

Using this option, a study and printing of stored Uroculture cases is possible. The stored data can also be modified at user's choice. The database can be queried by specifying the content of one or several fields in one search.

In the upper part of the window, the case number of the current data is shown. The total number of cases in the database (Example: Case 1 of 100) and the display mode are also reported.

The display mode refers to the way data was extracted from de database: if the option "Browse" was used, "All the cases" will be reported, but if a searching process was executed, "Search" will be reported and only those cases matching the search conditions will be available. The term "Viewing" informs if the displayed data corresponds to "General data" or to the final "Results" of the test.

Once "Database" has been selected, the following options will be available inside the window:
- **First:** Displays the first case of the database.
- **Previous:** Displays the case that is previous to the current.
- **Next:** Goes to the case that follows the current.
- **Last:** Jumps to the last case of the database.
- **Go to case:** Jumps to a selected case number.
- **Data/Results:** Switches the shown data from "General data" to test "Results".
- **Edit:** To edit the general data of the test.
- **Search/All Cases:** This option switches the viewing mode from the results of a "Search" process to "All cases" available in the database. Upon selecting the Search mode, a window will be opened where the operator can restrict 14 fields of the database in order to meet specified requirements of the query. Each option will supply a menu where the search criterion for the selected field can be chosen. Three commands can be used within this window:
   - **Search:** Allows to start a search or query according to a pre-defined set of field restrictions.
   - **New:** Formulates a new search or query under different restrictions.
   - **Return:** Returns to the previous window.

Some of the available "Search" criteria are now explained:
- **Containing:** Allows to search without taking into account if the field was typed in upper or lowercases. It also allows to search for words or syllables contained in a word or phrase, for example: "infect" in severe infection.
- **Not containing:** Performs in a similar way that the "containing" option with the difference that it will exclude those cases that meets the specified condition.
- **Included in list:** Allows to create a list with the desired requirements. This criterion is used while searching for several germs. Upon activation of this option, a list will be displayed where elements can be added or deleted.
- **Not included in list:** Similar to the "included in list" option but excluding the cases that meets the desired condition.
- **Ignore:** Ignores the selected field during the search process.
- **Exactly as and different from:** Compare exact words, considering if they where typed in upper or lowercases. This means those two identical words, one typed in uppercases and the other in lowercases will look different to this search option. To make a search that will not consider this condition, use the "containing" or "not containing" option as convenient.
- **Print:** Produces a printout to a specified printer or to an ASCII file.
- **Return:** Returns to the UROCULTURE MENU.

### Options:

Allows to set the following possibilities:
- Automatic printout after antibiogram measurement.
- Enable or disable the uroculture's time-out sound signal.
- The use of colors in the screen report.
- The time that will elapse between first and next uroculture readings. (4:00 hours is program's default)

### Compensate:

User choice to set the 0 McFarland reference for microbial growth measurements. The procedure is carry out using a vial containing culture medium un-inoculated.

### Consecutive:

Allows to set the consecutive number for the new cases of uroculture whenever the setting does not interfere with an existing unread uroculture.

This option becomes useful when it is required to start the uroculture's counter in a number different than 1, for instance, to start from 50, 100, etc.
*Note: The program handles the consecutive number as the "consecutive of the day." When a change of date is detected, the system, by default, will set the consecutive number to 1.*

### Help:

Shows user's information about the system and each of its options and operations.

### Return:

Returns to the MAIN MENU.

### Antibiogram Menu.

ATB: Antibiogram Menu.

Upon selecting the ATB icon in the Main Menu, an additional sub-menu with 12 basic operations required for this test will be supplied.

Option's description:

### Read ATB:

Allows the reading of an antibiogram that will determine the antibiotic susceptibility scheme of a given germ.

As a first step, the system will automatically check if the sensor has been rinsed and compensated and if the peristaltic pump is working properly. This procedure is transparent to the user if no problems are encountered. Any detected malfunction will be communicated to the user.

When the first ATB of a day is going to be executed, the system will proceed in order to compensate the electronic operation point of the microflow sensor. To comply with this requirement, a totally interactive step by step procedure will guide the operator through the process.

Once the adjustment is finished, the system will send a report with the "Compensation Constant", which is a numerical value that will be normally found below 2.00. However, values above 2.00 but fewer than 2.50 will be accepted and the user will be warn about the convenience of installing a new sensor.

In case all the technical requirements are fulfilled, an edition form, where the general data of the case may be typed, will be presented.

The following fields will become available:
- Case History
- First Name
- Middle name
- Last name
- Age
- Sex
- Test date
- Sample *
- Description
- Microorganism *
- Area *
- Doctor
- Nosocomial
- Deceased

Two commands are now available:
- **Cancel:** Returns to the ATB Menu.
- **OK:** Selecting this option will take the user to the measuring environment where its case number identifies each antibiogram. A low frequency tone signal, together with an "Insert Sensor" message in the window's title bar, will indicate to insert the sensor in the first well. A higher frequency tone and a "Remove Sensor" message will indicate that the corresponding measurement has been done and that the sensor should be removed from the current well and inserted in the following. This procedure will be repeated for each well in the strip. The antibiotic set in use and the case number will be displayed in this window. During measurements, the system constantly monitors any possible operation error, supplying instructions to solve any conflict that can arise.

The reading cycle is illustrated in the following diagram:
**READING CYCLE**
Message: **Insert sensor** + Low pitch tone => Sensor inside the well + Reading result
Message: **Remove sensor** + High pitch tone => Remove sensor from current well and jump to next well

During measurements, the following actions are available:
- **Previous:** This option allows to make a new reading of the well, which is previous to the current. It is used in order to correct reading errors.
- **Next:** Jumps to the next well. The current reading will be ignored.
- **Abort:** Cancels the current antibiogram measurement.

When the complete set has been read, a new window with the results of the antibiogram will be shown, where the germ's tolerance to each antibiotic is classified as **SENSITIVE, INTERMIDIATE** or **RESISTANT.** For each antibiotic under test, the inhibition percent is shown in units. The normalization algorithm will set to 1000 units the standard value of the negative control well.

Three additional parameters, the Minimum Allowed Growth, the Growth Index and the Inhibition Factor will be taken into account in order to classify the antibiograms results according to three possibilities:
- **Successful Antibiogram:** The measured data and the final results are reliable.
- **Not reliable Antibiogram:** The Minimum Allowed Growth has not been reached.

- **Useless Antibiogram:** A mathematical analysis of the Inhibition Factor shows an abnormal data set.

### URO-ATB:

Links a uroculture to an antibiogram. This option is used when an antibiogram of a positive urine sample is made in order to inherit the patient's data. To select the case, to which the test is going to be done, only the consecutive number and the date of the test are required. These cases are then identified by the designation URO-ATB and the corresponding consecutive number in the uroculture's database.

### McFarland:

Activates the McFarland calibrator for the monitoring of microbial growth.

### Database:

Allows to display and process the stored data of antibiogram cases. The procedure is similar to the one described in the "UROCULTURE MENU" (Database).

### Options:

Same as in the URO Menu. Allows to set the following possibilities:
- Automatic printout after antibiograms measurement.
- Enable or disable the urocultures time-out sound signal.
- The use of colors in the screen's report.
- The time that will elapse between first and next uroculture readings. (4:00 hours is program's default)

### Antibiotics:

Enables the operator to delete, modify or use an already existing set of antibiotics or to create a new one. The antibiotic scheme defined by the customer must match exactly the antibiotic discs existing in the plate or strip. Selecting the "Antibiotics" option, will bring the following possibilities:
- **New:** Allows to create a scheme for a new antibiotic set. Upon selection, the user will be asked for the number of strips and the number of wells per strip in order to build up the corresponding scheme (Normally 2 x 8). Afterwards, a window will be displayed in order to fill up the name of the set and to select, from a supplied list, each of the antibiotics that will be used.
- **View:** Allows to display or print a specific set of antibiotics.
- **Modify:** Changes the distribution or deletes antibiotics from a specific set.
- **Delete:** Deletes a complete set of antibiotics.
- **Make current:** This option predetermines the antibiotic set that will be used for the next antibiograms. To change the current setting, the desired set should be selected and the option "Make current" activated after the selection is done.
- **Select all:** Selects every existing antibiotic set
- **Unselect:** Unselect every antibiotic set previously selected.
- **Return:** Returns to the previous option.

### Stability:

This procedure is used to test the sensor's performance. Statistic parameters such as mean value, standard deviation and variance coefficient of a set of 16 identical samples are calculated. Results are stored in a specific database where they can be retrieved for viewing or printing at user's choice.

To perform this test, use distilled water for every one of the 16 measurements.

The expected values for these statistical parameters are:
2000 < Mean value: < 3000
Standard Deviation < 20
Variance coefficient < 2.00

### Clean Sensor:

In addition to the systematic daily cleaning procedures indicated automatically by the system's software, a user's choice Sensor Cleaning Procedure can be carried out in which the duration of each step can be manually programmed. The complete cleaning cycle is composed of three basic steps:
1) A Biologic detergent dilution is supplied to the flow sensor using the peristaltic pump.
2) Distilled water replaces the biologic detergent for rinsing the sensor.
3) A period of time is allowed to pass in order to flush the complete system.

### Calibrate:

Executes the calibration procedure for the microflow sensor. In order to update the system's performance, calibration must be done each time a new sensor is installed or with the periodicity indicated by the software.

Calibration is a procedure by means of which the response level of each sensor to a predetermined bacterial growth (0.5 McFarland) is established. It is not a daily routine and it is recommended to perform calibration each and every time that the software recommends this action in order to keep a standardized response and sensitivity.

To perform calibration, inoculate a flask with *Staphylococcus aureus* with a 0.5 McFarland Growth Index (C+) in accordance to the system's McFarland calibrator. Use a second sterile culture medium flask in order to obtain a 0 McFarland Index (C-).

Select "Calibrate" from the ATB Menu and follow the interactive procedure in order to perform three alternate readings of a (C+) followed by a (C-).

The system will calculate a mean value for the three measurements and the final result will be stored after selecting "OK".

### Flow Check:

This auxiliary procedure helps the operator while checking the amount and stability of the flow delivered by the peristaltic pump, since a lack of continuity or an incorrect flow may cause errors during the measurements.

The flow is checked using a 10 milliliter graduate probe and a recipient with distilled water.

To perform the check, insert both ends of the hose in a recipient filled with distilled water. Select the "Proceed" command, available inside the "Flow check" option of the ATB Menu. The interactive procedure will guide you through the whole process. Once finished, verify that the obtained flow is about of 2,4 ml/min.

If the expected volume is not reached, check the cassettes tension lever and the tension of the silicone hose within the cassette. Also check the technical conditions of the silicone hose, especially of the section inside the cassette which is in mechanical contact with the pump's rollers. If the hose is extremely collapsed or damaged, install a new one.

### Help:

Shows information about the system and each option.

### Return:

Returns to the Main Menu.

### Example 6: Results of the clinical trials carried out in Cuba:

A total of 567 urine samples were analyzed for the presence of significant number of uropathogens, using the system of the present invention, and matching the results with those obtained by reference method CLED (semiquantitative plate of culture method of Claridge). According to analysis 126 samples were positive by CLED, while 108 were positive using present system in just 4 hours, while CLED method results were ready after 24-48 hours after culture medium inoculation. The present system was effective in 86.1% for detection of positive samples in only 4 hours after samples inoculation.

From 441 samples found negatives by CLED method, the present system was able to detect 440 negatives in a period of 4 hours, for an affectivity of 99.8%. The general correlation between present system and the traditional CLED method was 89.1%.

### Example 7: Results of clinical trials carried out in Canada:

In total 1,016 urine samples have been investigated in a prospective manner. Results obtained with the system were compared with the semiquantitative plate of culture method of Claridge, used as the reference method for the detection of bacteriuria. For routine culture, 0.0001 ml of urine was delivered to a CLED agar plate using a calibrated disposable loop. This method detects≥ 1000colony forming units/ml (cfu/ml).

Turbidity readings were made at 2,3, 4 and 5 hours after inoculation. In all there were 184 samples with positive (≥0.4 McFarland units). The time distribution and correlation with routine culture of these samples are shown in Table 1.

**TABLE 1.**

| Time (hours) | Number detected | Correlation with Routine Culture % |
|---|---|---|
| 2 | 32 | 97 |
| 3 | 80 | 88 |
| 4 | 52 | 87 |
| 5 | 20 | 65 |
| All | 184 | 86.4 |

Thus, the overall sensitivity of the present system was 86.4%, and the specificity (i.e. the ability to detect truly negative samples as defined by routine culture) was 98.5%.

### BRIEF DESCRIPTION OF THE DRAWINGS:

**Figure 1** shows the integral scheme of the equipment of the present invention, in a general overview. As it is shown, this equipment is based on a turbidimetric reader of microflow (1), which is fed by a peristaltic pump (2) and adjusted to an electronic equipment of high sensibility (3) that detects turbidimetric changes of (1) through a measure method which allows using a group of algorithms to detect small turbidimetric variations and properly prosecute obtained data. It is formed by turbidimetric measurement circuit (4) connected through an interface card to a central processing unit (5). This unit receives all keyboard commands (6) and delivers the results in a display (7). It can detect smallest variations of turbidity that occur in the culture inoculated medium in the sample that will be tested.
**Figure 2** represents a detailed scheme of the internal structure belonging to the turbidimetric reader (1) of the Figure 1. The little mouth of the reader (8) is introduced in the sample to be tested which then circulates through it with help of the peristaltic pump (2) of the Figure 1. The measurement chamber is composed by a plastic tube (9) introduced in a glass capillary (10). The light pass (11) is the orifice diameter through which the light coming from the photoemitter (12) should cross over to arrive to the measurement chamber (9 and 10). The intensity of the luminous radiation, transmitted through the measurement chamber (9 and 10) will depend on turbidity grade of the sample and it is measured by a photodetector (13). The radiation produced by the photoemitter (12), is stabilized by means of an electronic loop of conventional automatic control.
**Figure 3** shows turbidimetric reader working through a diagram of flow. Firstly, the presence of the turbidimetric reader is verified by means of the subroutine of detection and if it is present, then the existence of the peristaltic pump is checked which is necessary for the turbidimetric reader working and for cleaning execution subroutine, besides it establishes the required working flow. Once all the working parameters has been regulated, the turbidimetric reader will be ready. Any subroutine that is infringed will disable the reader from functioning.
**Figure 4** shows the components of the diagnostic kit, constituted by a vial containing culture medium and the polymer, the strip support and the strip used for antibiogram determination of the sample to be tested.

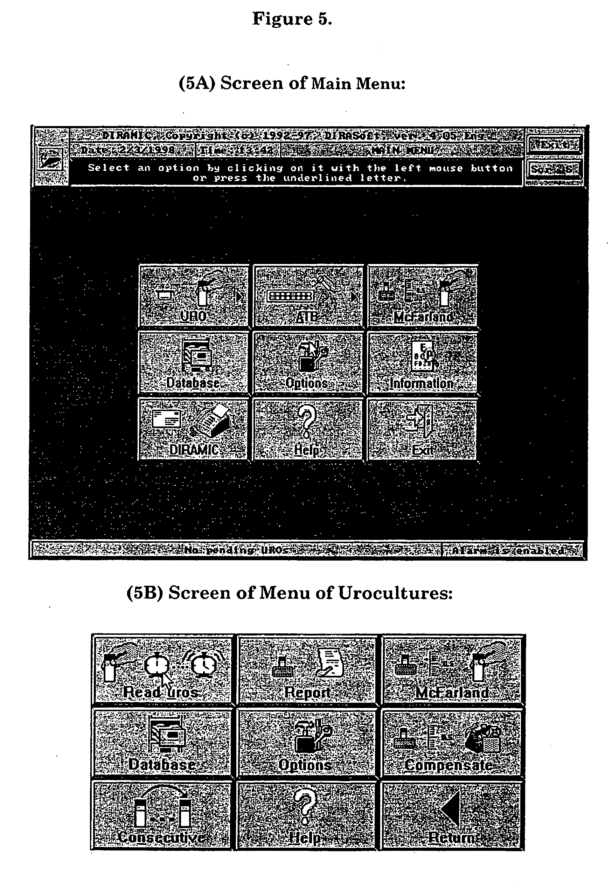

**Figure 5** shows two screens related to the program that follows the main procedures for the execution of the method subject of the present invention.

**Screen of Main Menu (5A)**: It constitutes the starting point for all operations that are performed when these are activated.

Nine icons define the available options within main menu, from where are controlled all the actions of the system. These are:
URO: Activates the icons menu of urocultures.
ATB: Activates the icons menu of the antibiogram.
McFarland: Permits to measure the content of the vial in the calibration unit (McFarland scale).
Data base: Permits to access to the data base of antibiograms or urocultures.
Options: To change the configuration of the system. Information: Shows administrative information of the system. DIRAMIC: Offers the address of the producing of the system. Help: Gives information on the current option.
Exit: To abandon the system.
**Screen of Menu of Urocultures (5B):** It represents a specific menu for the particular treatment of the urocultures, which contains the operations to perform during these tests, as a representation of the main options given by the Main Menu.

## Claims

1. Method for microbial diagnosis in urine, which comprises the following steps:
a) An aliquot of a urine sample directly obtained from its source is innoculated in a glass vial containing culture medium, a polymer and substrates MU-β-D-glucuronide and L-tryptophane, both solubilized in potassium phosphate 50 mM, pH 7 to 7.5,
b) The turbidity of said vial is determined to time = 0;
c) The vial is incubated between 2 and 6 hours to a temperature between 35 and 37° C;
d) The growth index is determined between time 0 and the selected time of incubation and samples positive are discriminated from negative ones in dependence of the increase of the turbidity, being positives those samples with increases higher than 0.08 McFarland units, from which aliquots are taken for further steps;
e) an aliquot of a positive sample is taken from step d) and it is transferred to a new glass vial containing fresh culture medium, which is dispensed in a volume of 200 µl in each microwell of a non-transparent strip containing two free positions for negative and positive controls, which are filled with only culture medium and inoculated culture medium respectively, and 10 to 22 positions in which antibiotic discs are organized, and it is incubated between 3 to 4 hours to a temperature between 35 and 37° C;
f) the strip is read by placing a turbidimetric reader of microflow, which is fed by a peristaltic pump, in each microwell following the instructions emitted by a computer program package for data acquisition and processing and creation of databases designed for that purpose, wherein the turbidimetric reader of microflow comprises a photoemitter, a photodetector, and a measurement chamber, wherein said measurement chamber is composed of a plastic tube introduced in a glass capillary, the measurement chamber being illuminated by a light emitted from said photoemitter and passing through said measurement chamber;
g) from the density values obtained, the growth index is calculated in the controls, and the inhibitions percentages are calculated for each sample for each antibiotic;
h) according to the inhibition level shown by the samples, the criteria of sensitivity, resistant or intermediate are adjudicated, among inhibition values in a range of 60 to 100%, wherein samples with inhibition percentage smaller than 60% can be considered resistant, those showing values between 60 to 80% can be considered sensitive in an intermediate level and those which are inhibited between 80 and 100% are considered sensitive to the antibiotic tested;
i) an aliquot taken from the step d) is submitted to a source of U.V. light, in order to detect the fluoresce generated due to 4-methylumbeliferone liberation, and developing the Indol formation using a modified Kovacks reagent, constituted by 2 g of paradimethylaminobenzaldehyde diluted in ethanol and 20 ml of concentrated hydrochloric acid, for detection of MU- beta -D-glucuronidase and tryptophanase enzymatic activity produced by E.coli;
j) for those samples wherein E.coli was not identified in the step i), the aliquot previously obtained in the step d) is submitted to the traditional process of isolation and identification.

2. The method according to claim 1, wherein the culture medium is modified Mueller-Hinton broth OXOID.

3. Equipment suitable for carrying out antibiogram determination in steps f) to h) of the method according to claim 1 or 2 comprising:
(1) a turbidimetric reader of microflow (1) comprising a photoemitter (12), a photodetector (13), and a measurement chamber (9 and 10), wherein said measurement chamber is composed of a plastic tube (9) introduced in a glass capillary (10), the measurement chamber being illuminated by a light emitted from said photoemitter and passing through said measurement chamber;
(2) a nontransparent strip containing microwells;
(3) a peristaltic pump (2) for circulating a sample through the measurement chamber of the turbidimetric reader of microflow; and a computer (5) having a program package for data acquisition and processing and creation of databases;
wherein
the turbidimetric reader of microflow is connected to the computer by an interface card and
said turbidimetric reader of microflow includes a mouth part (8) suitable for introduction in a sample in a microwell of the nontransparent strip to be tested by circulating a sample through said measurement chamber by action of said peristaltic pump.

4. The equipment according to claim 3, wherein the non-transparent strips include two free positions for positive and negative controls, and also another 10 to 22 positions where antibiotic discs can be placed according to the user's option.

5. The equipment according to claim 4, wherein the non-transparent strips include antibiotic discs.

## Patentansprüche

1. Verfahren zur mikrobiellen Diagnose im Urin, welches die folgenden Schritte umfasst:
a) ein Aliquot einer Urinprobe, direkt erhalten aus ihrer Quelle, wird verimpft in einer Glasphiole, die Kulturmedium, ein Polymer und die Substrate MU-β-D-Glucuronid und L-Tryptophan, die beide löslich gemacht wurden in Kalium-Phosphat 50 mM, pH 7 bis 7,5, enthält;
b) wobei die Trübung der besagten Phiole bestimmt wird zum Zeitpunkt = 0;
c) die Phiole wird zwischen 2 und 6 Stunden bei einer Temperatur zwischen 35 und 37°C; inkubiert
d) der Wachstumsindex wird bestimmt zwischen Zeitpunkt 0 und der ausgewählten Zeit der Inkubation, und Proben, die positiv sind, werden diskriminiert gegen die negativen Proben in Abhängigkeit der Zunahme der Trübung, wobei die positiven diejenigen Proben sind mit Zuwächsen von mehr als 0,08 McFarland-Einheiten, von welchen Aliquots genommen werden für weitere Schritte;
e) ein Aliquot einer positiven Probe wird aus Schritt d) genommen und transferriert in eine neue Glasphiole, die frisches Kulturmedium enthält, das verteilt wird in einem Volumen von 200 µl in jedes Mikrowell eines nicht-transparenten Streifens, der zwei freie Positionen für negative und positive Kontrollen enthält, die nur mit Kulturmedium bzw. mit verimpften Kulturmedium befüllt werden, sowie 10 bis 22 Positionen, in welchen Antibiotikum-Scheiben organisiert sind, und es wird inkubiert zwischen 3 bis 4 Stunden bei einer Temperatur zwischen 35 und 37°C;
f) der Streifen wird gelesen durch Platzieren eines Trübungs-Lesegerätes für Mikrofluss, der gespeist wird durch eine peristaltische Pumpe, in jedem Mikrowell, folgend den Instruktionen, die durch ein Computer-Programm-Paket ausgegeben werden für Datenzugriff und Verarbeitung bzw. das Erzeugen von Datenbanken, die konzipiert sind für diese Zweck, wobei das Trübungslesegerät für Mikrofluss einen Photoemitter enthält, einen Photodetektor sowie eine Messzelle, wobei besagte Messzelle aus einem Plastikröhrchen besteht, eingebracht in eine Klaskapillare, und die Messkammer illuminiert wird durch ein Licht, das von besagtem Photoemitter emittiert wird und durch besagte Messzelle tritt;
g) aus den Dichtewerten, die erhalten werden, wird der Wachstumsindex berechnet in den Kontrollen und die Inhibitions-Prozentsätze werden berechnet für jede Probe für jedes Antibiotikum;
h) gemäß dem Inhibitionsgrad, gezeigt durch die Proben, werden Kriterien der Sensitivität, Resistenz oder dazwischen liegend unter lnhibitionswerten zugeordnet, die in einem Bereich von 60 bis 100% liegen, wobei Proben mit einem Inhibitions-Prozentsatz von weniger als 60% als resistent betrachtet werden können, diejenigen, die Werte zwischen 60 und 80% zeigen, als sensitiv auf einem Zwischenniveau betrachtet werden können, und diejenige die zwischen 80 und 100% inhibiert werden, als sensitiv auf das untersuchte Antibiotikum getestet betrachtet werden können;
i) ein Aliquot, das aus dem Schritt d) genommen wurde, wird einer UV-Lichtquelle ausgesetzt, um die Fluoreszenz nachzuweisen, die aufgrund der Freisetzung von 4-Methylumbeliferon bzw. der Entwicklung der Indol-Bildung unter Verwendung des modifizierten Kovacks-Reagenz erzeugt wird, das aus 2 g an Paradimethylamino-Benzaldehyd, verdünnt in Ethanol und 20 ml an konzentrierter Salzsäure besteht, zum Nachweis von MU-Beta-D-Glucuronidase- und Tryptophanase Enzym-Aktivität, erzeugt durch *E. coli;*
j) für diejenigen Proben, wo *E. coli* nicht in Schritt i) identifiziert wurde, wird das Aliquot, das zuvor in Schritt d) erhalten wurde, dem traditionellen Prozess der Isolierung und Identifikation unterzogen.

2. Das Verfahren gemäß Anspruch 1, wobei das Kulturmedium modifizierte Mueller-Hinton OXOID-Brühe ist.

3. Ein Equipment, das geeignet ist zum Durchführen einer Antibiogramm-Bestimmung in den Schritten f) bis h) des Verfahrens entsprechend den Ansprüche 1 oder 2, umfassend:
(1) ein Trübungslesegerät für Mikrofluss (1) umfassen ein Fotoemitter (12), einen Fotodetektor (13) sowie eine Messzelle (9) und (10), wobei besagte Messzelle aus einem Plastikrohr (9) besteht, das eingebracht ist in eine Glaskapillare (1 p), wobei die Messzelle illuminiert wird durch ein Licht, das emittiert wird von besagtem Fotoemitter und durch besagte Messzelle tritt;
(2) einen nicht-transparenter Streifen, der Mikrowells enthält;
(3) eine peristaltische Pumpe zum Zirkulieren einer Probe durch die Messzelle des Trübungslesegeräts für Mikrofluss; und
(4) einem Computer (5) mit einem Programmpaket für Datenzugriff sowie Verarbeitung bzw. Erzeugen von Datenbanken;
wobei
das Trübungslesegerät für Mikrofluss verknüpft ist mit dem Computer durch eine Schnittstellen-Karte und
besagtes Trübungslesegeräts für Mikrofluss einen Eingangsteil (8) aufweist, der geeignet ist zum Einbringen einer Probe in ein Mikrowell des nicht-transparenten Streifens, die untersucht werden soll durch Zirkulieren einer Probe durch besagte Messzelle mit Hilfe der Wirkung von besagter peristaltischer Pumpe.

4. Die Ausrüstung entsprechend Anspruch 3, wobei die nicht-transparenten Streifen zwei freie Positionen für positive und negative Kontrollen enthalten und auch weitere 10 bis 22 Positionen, wo Antibiotikum-Scheiben entsprechend dem Bedarf des Anwenders platziert werden können.

5. Die Ausrüstung gemäß Anspruch 4, wobei die nicht-transparenten Streifen Antibiotikums-Scheiben einschließen.

## Revendications

1. Procédé de diagnostic microbien dans l'urine, qui comprend les étapes suivantes :
a) une fraction aliquote d'un échantillon d'urine directement obtenu depuis sa source est inoculée dans un flacon en verre contenant un milieu de culture, un polymère et les substrats MU-13-17-glucuronide et L-tryptophane, tous deux solubilisés dans du phosphate de potassium 50 mM, pH 7 à 7,5 :
b) la turbidité dudit flacon est déterminée au temps = 0 ;
c) le flacon est incubé entre 2 et 6 heures à une température comprise entre 35 et 37 °C ;
d) l'indice de croissance est déterminé entre le temps 0 et le temps d'incubation choisi et les échantillons positifs sont différenciés des échantillons négatifs d'après l'augmentation de turbidité, les échantillons positifs étant ceux présentant une augmentation supérieure à 0,08 unités McFarland, échantillons desquels des fractions aliquotes sont prélevées pour les étapes suivantes ;
e) une fraction aliquote d'un échantillon positif est prélevée dans l'étape d) et est transférée dans un nouveau flacon en verre contenant du milieu de culture frais, qui est réparti par volume de 200 µl dans chaque micropuits d'une bande non transparente contenant deux positions libres pour les témoins positif et négatif, qui ne sont remplis que de milieu de culture et de milieu de culture inoculé, respectivement, et 10 à 22 positions dans lesquelles des disques antibiotiques sont placés, et la bande est incubée pendant 3 à 4 heures à une température comprise entre 35 et 37 °C ;
f) la bande est lue en plaçant un lecteur turbidimétrique de microflux, qui est alimenté par une pompe péristaltique, dans chaque micropuits en suivant les instructions émises par une logiciel informatique pour l'acquisition de données et le traitement et la création de bases de données conçu dans cet objectif, le lecteur turbidimétrique de microflux comprenant un photoémetteur, un photodétecteur, et une chambre de mesure, ladite chambre de mesure étant composée d'un tube de plastique introduit dans un capillaire en verre, la chambre de mesure étant illuminée par une lumière émise par ledit photoémetteur et passant à travers ladite chambre de mesure ;
g) d'après les valeurs de masse volumique obtenues, l'indice de croissance est calculé dans les témoins, et les pourcentages d'inhibition sont calculés pour chaque échantillon pour chaque antibiotique ;
h) selon le niveau d'inhibition présenté par les échantillons, les critères sensible, résistant ou intermédiaire sont attribués, dans l'intervalle des valeurs d'inhibition de 60 à 100 %, les échantillons présentant un pourcentage inférieur à 60 % pouvant être considérés comme résistants, ceux présentant une valeur comprise entre 60 et 80 % pouvant être considérés comme sensibles à un niveau intermédiaire et ceux qui sont inhibés entre 80 et 100 % étant considérés comme sensibles à l'antibiotique testé ;
i) une fraction aliquote prélevée dans l'étape d) est soumise à une source de lumière UV, afin de détecter la fluorescence générée par la libération de 4-mëthylombelïférone, et le développement de la formation d'indol en utilisant un réactif de Kovacks, constitué de 2 g de paradiméthylaminobenzaldéhyde dilué dans l'éthanol et 20 ml d'acide chlorhydrique concentré, pour la détection d'activités enzymatiques MU-bêta-D-glucuronidase et tryptophanase produites par E. coli ;
j) pour les échantillons dans lesquels E. coli n'a pas été identifiée dans l'étape i), la fraction aliquote préalablement obtenue dans l'étape d) est soumise au procédé classique d'isolement et d'identification.

2. Procédé selon la revendication 1, dans lequel le milieu de culture est du bouillon Mueller-Hinton OXOID modifié.

3. Equipement adapté à la mise en oeuvre de la détermination de l'antibiogramme dans les étapes f) à h) du procédé selon la revendication 1 ou 2, comprenant :
(1) un lecteur turbidimétrique de microflux (1) comprenant un photoémetteur (12), un photodétecteur (13), et une chambre de mesure (9 et 10), ladite chambre de mesure étant composée d'un tube de plastique (9) introduit dans un capillaire en verre (10), la chambre de mesure étant illuminée par une lumière émise par ledit photoémetteur et passant à travers ladite chambre de mesure ;
(2) une bande non transparente contenant des micropuits ;
(3) une pompe péristaltique (2) pour faire circuler un échantillon à travers la chambre de mesure du lecteur turbidimétrique de microflux ; et
(4) un ordinateur (5) muni d'un logiciel pour l'acquisition de données et le traitement et la création de bases de données ;
dans lequel
le lecteur turbidimétrique de microflux est connecté à l'ordinateur par une carte interface et
ledit lecteur turbidimétrique de microflux comprend une embouchure (8) appropriée à l'introduction dans un échantillon dans un micropuits de la bande non transparente à tester en faisant circuler un échantillon à travers ladite chambre de mesure par l'action de ladite pompe péristaltique.

4. Equipement selon la revendication 3, dans lequel les bandes non transparentes comprennent deux positions libres pour les témoins positif et négatif, et également 10 à 22 autres positions dans lesquelles des disques antibiotiques peuvent être placés au choix de l'utilisateur.

5. Equipement selon la revendication 3, dans lequel les bandes non transparentes comprennent des disques antibiotiques
